# EUROPEAN PATENT APPLICATION

(11) **EP 4 445 929 A1**
(43) Date of publication of application: **16.10.2024**
(21) Application number: 23167934.1
(22) Date of filing: 14.04.2023
(51) Int. Cl.: A61M 5/315, A61M 5/31

(54) **CONNECTED DRUG DELIVERY DEVICE**

(71) Applicant: Becton, Dickinson and Company, Franklin Lakes, NJ 07417-1880 (US)
(72) Inventor: MONCHOIX, Hervé, 38330 Biviers (FR); MARÉCHAL, Damien, 38640 Claix (FR)
(74) Representative: Germain Maureau

(57) **Abstract**

A plunger device for a drug delivery device is disclosed. The plunger device includes a plunger rod having a proximal end and a distal end configured to be attached to a stopper. The plunger device further includes at least one tracking element operatively connected to an outer surface of the plunger rod, the at least one tracking element extending along a longitudinal axis of the plunger rod. The plunger device also includes at least one power supply operatively connected to the at least one tracking element, and at least one current sensing device operatively connected to the at least one power supply and configured to detect an electrical contact established between the at least one tracking element and a conductive element external to the plunger rod.

## Description

### BACKGROUND OF THE INVENTION

### Field of the Invention

The present disclosure relates generally to drug delivery devices and components therefor, and in particular, to components allowing for detection and communication of the injection status of drug delivery devices.

The present disclosure also relates generally to systems for detecting initiation and cessation of drug delivery with a drug delivery device, as well as the progress of drug delivery, communicating such drug delivery information to a mobile device associated with a user of the drug delivery device.

### Description of Related Art

Drug delivery devices have traditionally lacked features that would allow a healthcare provider to automatically record or capture an injection status of drug delivery device to confirm appropriate administration of medicaments. Ensuring timely, appropriate administration of some medicaments can be critical, particularly, for example, in the instance of the evaluation of new drugs in clinical trials, where accurate information is essential.

While some devices provide the ability to communicate certain types of information, for example as described in International Patent Application Publication Nos. WO 2016/087512, WO 2017/070391, WO 2018/111969, WO 2018/213837, and WO 2021/094797, and in U.S. Patent Application Publication Nos. 2019/0083708, 2019/0321555, and 2019/0344019, a need exists in the art for a cost-effective device that accurately detects both initiation and cessation of drug delivery, as well as the current progress of drug delivery from the drug delivery device, and can communicate this drug delivery information to appropriate stakeholders, in a timely fashion.

### SUMMARY OF THE INVENTION

In accordance with an aspect of the present invention a plunger device for a drug delivery device includes a plunger rod having a proximal end and a distal end configured to be attached to a stopper, at least one tracking element operatively connected to an outer surface of the plunger rod, the at least one tracking element extending along a longitudinal axis of the plunger rod, at least one power supply operatively connected to the at least one tracking element, and at least one current sensing device operatively connected to the at least one power supply and configured to detect an electrical contact established between the at least one tracking element and a conductive element external to the plunger rod.

In accordance with another aspect of the invention, the plunger rod is configured to be used with a manual syringe.

In accordance with another aspect of the invention, the proximal end of the plunger rod includes a housing having a proximal end configured to be contacted by a user's thumb during drug delivery, a distal end attached to the proximal end of the plunger rod, and a sidewall therebetween defining an interior.

In accordance with another aspect of the invention, the proximal end of the housing is displaceable relative to the distal end of the housing when a distally-directed force is applied by the user during drug delivery.

In accordance with another aspect of the invention, the at least one tracking element includes two tracking elements positioned on the outer surface of the plunger rod opposite one another.

In accordance with another aspect of the invention, the at least one tracking element is a conductive strip.

In accordance with another aspect of the invention, the at least one power supply includes a battery.

In accordance with another aspect of the invention, the plunger device further includes a force sensor configured to identify a pressure applied to the proximal end of the plunger rod.

In accordance with another aspect of the invention, the at least one tracking element extends from the proximal end of the plunger rod to the distal end of the plunger rod.

In accordance with another aspect of the invention, the plunger device further includes a communication interface for issuing a signal when the electrical contact has been established between the at least one tracing element and the conductive element external to the plunger rod.

In accordance with an aspect of the invention, a drug delivery device includes a proximal end, a distal end, and a barrel therebetween defining an interior configured to receive a medicament. The device further includes a plunger device received at least partially within the interior. The plunger device includes a plunger rod having a proximal end and a distal end configured to be attached to a stopper, at least one tracking element operatively connected to an outer surface of the plunger rod, the at least one tracking element extending along a longitudinal axis of the plunger rod, at least one power supply operatively connected to the at least one tracking element, and at least one current sensing device operatively connected to the at least one power supply and configured to detect an electrical contact established between the at least one tracking element and a conductive element external to the plunger rod.

In accordance with another aspect of the invention, the drug delivery device includes a backstop operatively connected to a finger flange provided on the proximal end of the drug delivery device, the backstop comprising the conductive element external to the plunger rod.

In accordance with another aspect of the invention, the drug delivery device includes a ring operatively connected to the drug delivery device and configured to receive the plunger rod, the ring comprising the conductive element external to the plunger rod.

In accordance with another aspect of the present invention, a method of monitoring an injection procedure of a drug delivery device includes providing a drug delivery device. The drug delivery device includes a proximal end, a distal end, and a barrel therebetween defining an interior configured to receive a medicament. The device further includes a plunger device received at least partially within the interior. The plunger device includes a plunger rod having a proximal end and a distal end configured to be attached to a stopper, at least one tracking element operatively connected to an outer surface of the plunger rod, the at least one tracking element extending along a longitudinal axis of the plunger rod, at least one power supply operatively connected to the at least one tracking element, and at least one current sensing device operatively connected to the at least one power supply and configured to detect an electrical contact established between the at least one tracking element and a conductive element external to the plunger rod. The method further includes receiving, with the at least one current sensor device, a first signal indicating that the electrical contact between the at least one tracking element and the conductive element external to the plunger rod has been established. The method further includes transmitting, with at least one communication interface, the first signal to a remote device, and receiving, with the at least current sensing device, a second signal indicating a resistance of an electrical circuit established between the at least one tracking element and the conductive element external to the plunger rod. The method further includes transmitting, with the at least one communication interface, the second signal to the remote device.

In accordance with another aspect of the invention, the at least one current sensing device is configured to receive signals of the resistance of the electrical circuit during the entire injection procedure.

### BRIEF DESCRIPTION OF THE DRAWINGS

**FIGS. 1A and 1B** are perspective (FIG. 1A) and side (FIG. 1B) views of a drug delivery device according to a non-limiting embodiment or aspect described herein;
**FIG. 2** is a perspective view of a plunger device according to a non-limiting embodiment or aspect described herein;
**FIG. 3** is a perspective view of a plunger device according to a non-limiting embodiment or aspect described herein;
**FIG. 4** is a diagram of a non-limiting embodiment or aspect of an environment in which systems, devices, and/or methods described herein may be implemented;
**FIG. 5** is a diagram of a non-limiting embodiment or aspect of components of one or more devices of **FIG. 4****;**
**FIG. 6** is a side view of a drug delivery device according to a non-limiting embodiment or aspect described herein shown in a pre-injection position;
**FIG. 7** is a side view of the drug delivery device of **FIG. 6** shown in a mid-injection position;
**FIG. 8** is a side view of the drug delivery device of **FIG. 6** shown in a post-injection position;
**FIG. 9** is a schematic illustration of a communication module of the drug delivery device of **FIG. 6****;** and
**FIG. 10** is a cross-sectional view of a backstop of the drug delivery device of **FIG. 6****.**

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible.. Any known electronic communication protocols and/or algorithms can be used such as, for example, TCP/IP (including HTTP and other protocols), WLAN (including 902.11a/b/g/n and other radio frequency-based protocols and methods), analog transmissions, Global System for Mobile Communications (GSM), 3G/4G/LTE, BLLTETOOTH, NFC, RFID, ZigBee, EnOcean, TransferJet, Wireless USB, and the like known to those of skill in the art. In some non-limiting embodiments, a message may refer to a network packet (e.g., a data packet and/or the like) that includes data.

As used herein, the term "computing device" may refer to one or more electronic devices configured to process data. A computing device may, in some examples, include the necessary components to receive, process, and output data, such as a processor, a display, a memory, an input device, a network interface, and/or the like. A computing device may be a mobile device. As an example, a mobile device may include a cellular phone (e.g., a smartphone or standard cellular phone), a portable computer, a wearable device (e.g., watches, glasses, lenses, clothing, and/or the like), a personal digital assistant (PDA), and/or other like devices. A computing device may also be a desktop computer or other form of non-mobile computer.

Provided herein are a plunger for a drug delivery device, a drug delivery device including such a plunger, and systems and methods including use of such a plunger, for monitoring delivery of a medicament with the drug delivery device. The devices, systems, and methods described herein improve upon traditional devices and methods of determining administration of medicaments, by enabling timely and accurate monitoring of drug delivery by relevant stakeholders in a cost-effective manner, improving, for example, clinical trials for newly-developed medicaments.

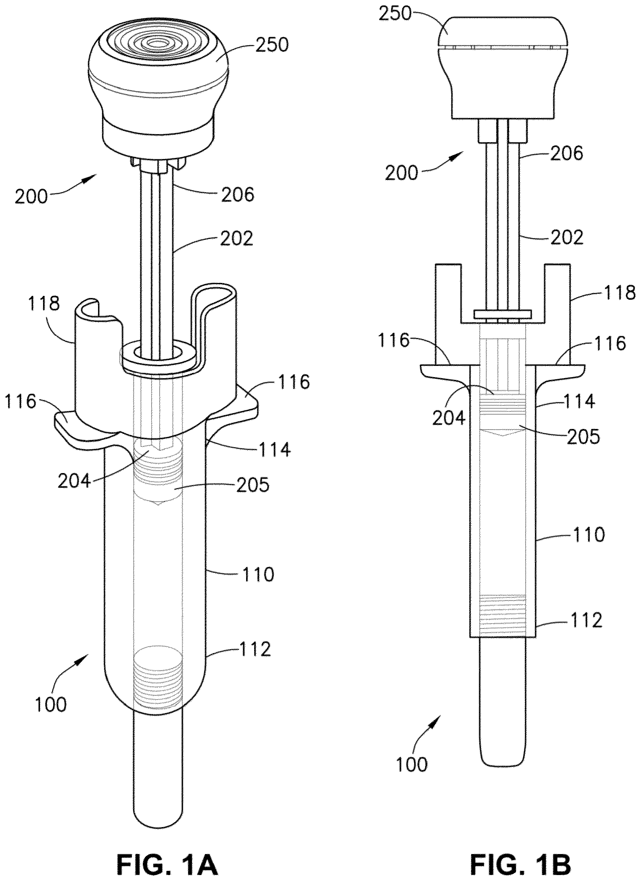

Referring to FIGS. 1A and 1B, shown is a drug delivery device 100 with a plunger device 200. While a manual syringe is exemplified in FIG. 1, those of skill in the art will appreciate that the components of the plunger device 200 can be utilized in any type of drug delivery device, including autoinjectors, wearable injectors, pump devices, and the like. The drug delivery device 100 exemplified in FIGS. 1A and 1B includes a distal end 112, proximal end 114, and barrel 110 therebetween defining an interior configured to hold one or more medicaments.

Drug delivery device 100 can be formed of any suitable material, including glass or plastics. Barrel 110 can be coated with any known coating(s), as appropriate to decrease friction between components and/or reduce interactions between the material of barrel 110 and any medicaments held within drug delivery device 100, including polydimethylsiloxane-based coatings that are optionally crosslinked. Coatings for decreasing friction between a stopper and sidewall and/or for reducing interactions between the sidewall and the medicament, for example siloxane-based coatings, are known to those of skill in the art, and are described in, for example, U.S. Patent Application Publication Nos. 2014/0221934 and 2014/0110297, the contents of which are incorporated herein by reference in their entirety.

In non-limiting embodiments or aspects, drug delivery device 100 includes a needle (not shown) at a distal end thereof. Suitable needles can be of any useful gauge, can be formed of any suitable material, and can be fixed to distal end 112 of drug delivery device 100, or can be removable. Drug delivery device 100 can include any number of suitable safety features, including a rigid needle shield (RNS) for protecting any needles that may be included, a cap 115, and/or a safety device 118, such as that shown in FIGS. 1A and 1B. RNSs are known in the art, and can include an inner, elastomeric portion that encases an injection needle, and an outer, rigid plastic portion. Suitable cap assemblies are described in, for example, International Patent Application Publication No. WO 2020/173998, the content of which is incorporated herein by reference in its entirety. Suitable safety devices 118 can include those that prevent reuse of the syringe, those that shield any needles before and/or after delivery, and the like, as are known to those of skill in the art. For example, safety devices are described in U.S. Patent No. 10,702,663, and are available commercially from Becton Dickinson and Company under the tradename UltraSafe. As will be described, in non-limiting embodiments or aspects, safety device 118 can interact with plunger device 200 to indicate end of drug delivery.

Returning to FIGS. 1A and 1B, drug delivery device 100 can include at proximal end 114 thereof one or more finger flange(s) 116, for assisting a user in gripping drug delivery device 100. As will be described, in non-limiting embodiments or aspects, one or more finger flange(s) 116 can interact with plunger rod 200 to indicate end of drug delivery.

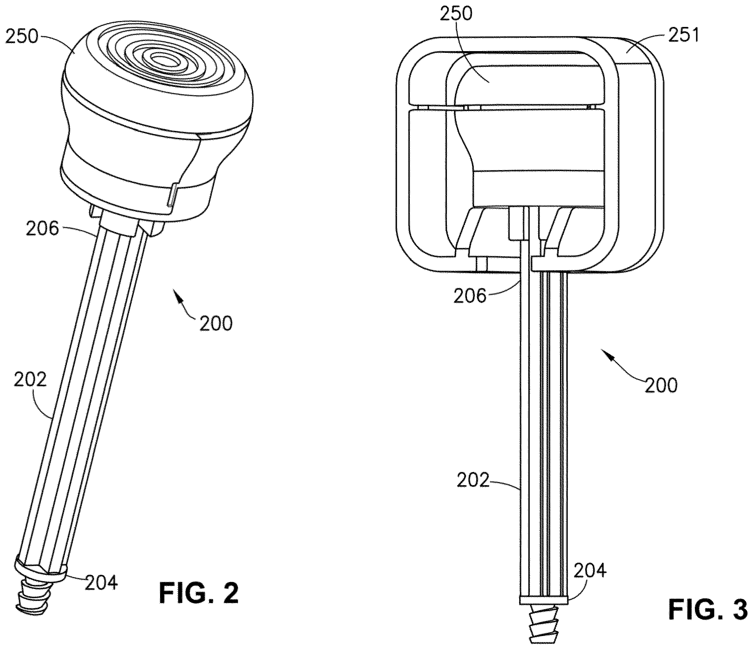

Referring to FIGS. 1A-3, plunger device 200 can include a plunger rod 202 extending between a distal end 204 and a proximal end 206. Distal end 204 of plunger rod 202 can be configured to interact, detachably or fixedly, with a stopper 205. Stopper 205 can be formed of any material, such as elastomeric materials, known to those in the art to be suitable, such as plastics, natural rubbers, synthetic rubbers, and the like. In non-limiting embodiments or aspects, a useful stopper is formed of a natural or synthetic rubber. In non-limiting embodiments or aspects, the rubber is butyl rubber (IIR), isoprene rubber (IR), butadiene rubber (BR), styrene-butadiene rubber (SBR), ethylene-propylene rubber (EPM), ethylene-propylene-diene rubber (EPDM), chlorosulphonated polyethylene (CSM), ethylene-vinyl acetate copolymer (EVA), styrene-isoprene rubber (SIR), thermoplastic elastomers, and/or natural rubbers. In non-limiting embodiments or aspects, the stopper can be formed of an elastomeric copolymer, including, without limitation, thermoplastic elastomers, thermoplastic vulcanizates, styrene copolymers such as styrene-butadiene (SBR or SBS) copolymers, styrene-isoprene (SIS) block polymers, or styrene-isoprene/butadiene (SIBS).

Stopper 205 can optionally be coated with any known coating(s), as appropriate, including polydimethylsiloxane-based coatings that are optionally crosslinked. Coatings for decreasing friction between a stopper and sidewall and/or for reducing interactions between the material of the stopper and the medicament, for example siloxane-based coatings, are known to those of skill in the art, and are described in, for example, U.S. Patent Application Publication Nos. 2014/0221934 and 2014/0110297, the contents of which are incorporated herein by reference in their entirety.

Plunger device 200 can include at proximal end 206 thereof a connection module 250, having a housing 252 having a proximal end 254 and a distal end 256 defining an interior. Connection module housing 252 can be formed of any suitable material, including plastics. In non-limiting embodiments or aspects, proximal end 254 of housing 252 includes a displaceable thumb pad 258, such that when a user applies a distally-directed force thereto during a drug delivery procedure, thumb pad 258 and/or proximal end 254 of housing 252 is displaced distally. As will be described, proximal end 254 of housing 252 can interact with a switch within housing 252 to indicate initiation of drug delivery.

In non-limiting embodiments or aspects, plunger device 200 can include a case 251, to protect connection module 250 and, for example, prevent a force from acting on thumb pad 258 and/or proximal end 254 of housing 252 and inadvertently displacing proximal end 254 of housing 252.

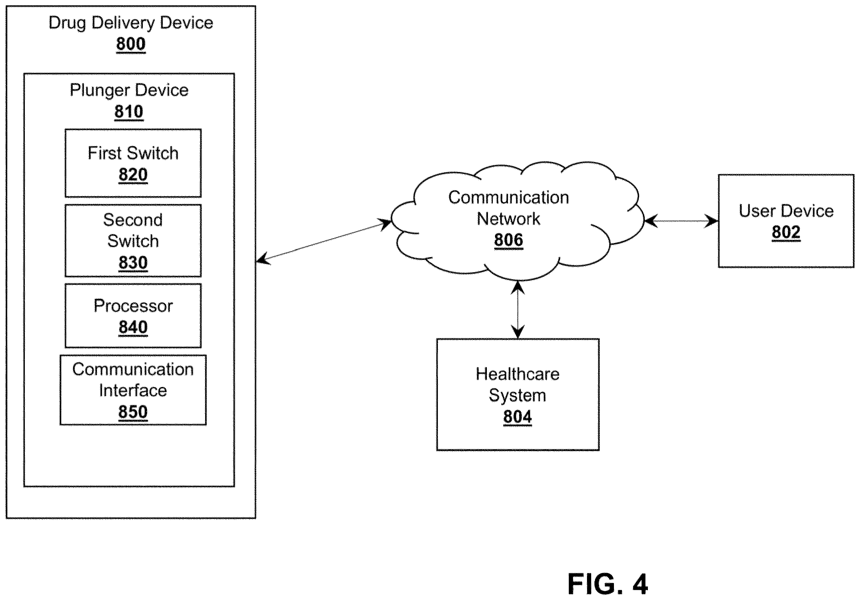

Referring now to FIG. 4, FIG. 4 is a diagram of an example environment in which devices, systems, and/or methods, described herein, may be implemented. As shown in FIG. 4, the environment can include drug delivery device 800, user device 802, healthcare system 804, and/or communication network 806. Drug delivery device 800, user device 802, and healthcare system 804 may interconnect (e.g., establish a connection to communicate) via wired connections, wireless connections, or a combination of wired and wireless connections.

Drug delivery device 800, which can be a syringe, autoinjector, wearable injector, and/or pump as described herein, can include, as described above, plunger rod 810 including first switch 820, second switch 830, processor 840, and communication interface 850. As described above, drug delivery device 800 can be configured to communicate with a user device 802, such as a computing device as described herein.

Communication network 806 may include one or more wired and/or wireless networks. For example, communication network 806 may include a BLLTETOOTH connection (e.g., between drug delivery device 800 and user device 802), a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN) and/or the like), a private network, an ad hoc network, an intranet, the Internet, a fiber optic-based network, a cloud computing network, and/or the like, and/or a combination of some or all of these or other types of networks.

User device 802 can be a computing device as described herein, in some non-limiting embodiments or aspects, a smartphone. User device 802 can be programmed or configured to communicate, for example through communication network 806, with a healthcare system 804, for example through a mobile application executable on user device 802.

Healthcare system 804 may include a server, a group of servers, and/or other like devices. More than one healthcare system 804 can be provided, for example, a system associated with a device manufacturer, a system associated with a pharmaceutical manufacturer, a system associated with a healthcare provider, a system associated with clinical research, a system associated with a government agency, and/or a system associated with a study sponsor, for example a sponsor of a clinical trial.

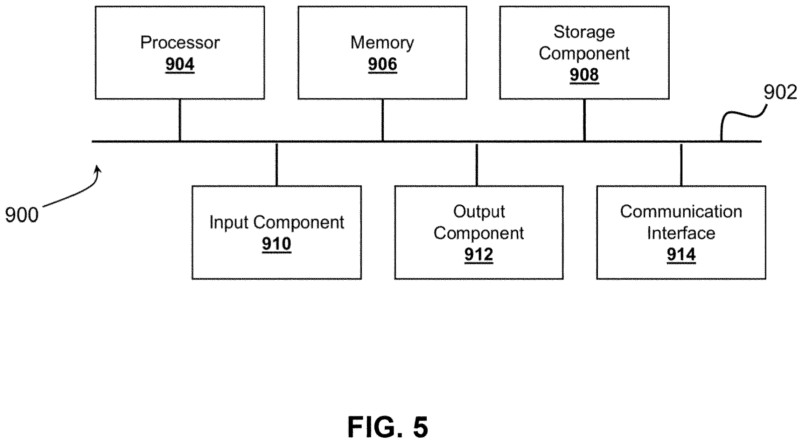

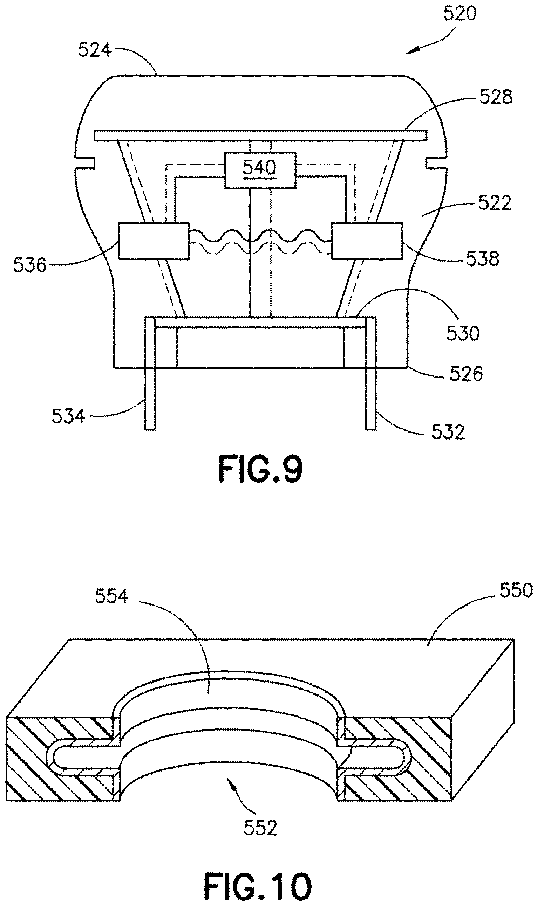

Referring now to FIG. 5, shown is a diagram of example components of an exemplary computing device 900 that can be used in the systems, devices, and methods described herein. Such a computing device 900 may correspond to component within a connection module of a drug delivery device as described herein, a user device as described herein, and/or a healthcare system as described herein. As shown in FIG. 9, a computing device 900 may include bus 902, processor 904, memory 906, storage component 908, input component 910, output component 912, and/or communication interface 914.

Bus 902 may include a component that permits communication among the components of computing device 900. In some non-limiting embodiments, processor 904 may be implemented in hardware, software, or a combination of hardware and software. For example, processor 904 may include a processor (e.g., a central processing unit (CPU), a graphics processing unit (GPU), an accelerated processing unit (APU), and/or the like), a microprocessor, a digital signal processor (DSP), and/or any processing component (e.g., a field-programmable gate array (FPGA), an application-specific integrated circuit (ASIC), and/or the like) that can be programmed to perform a function. Memory 906 may include random access memory (RAM), read-only memory (ROM), and/or another type of dynamic or static storage memory (e.g., flash memory, magnetic memory, optical memory, and/or the like) that stores information and/or instructions for use by processor 904.

Storage component 908 may store information and/or software related to the operation and use of computing device 900. For example, storage component 908 may include a hard disk (e.g., a magnetic disk, an optical disk, a magneto-optic disk, a solid state disk, and/or the like), a compact disc (CD), a digital versatile disc (DVD), a floppy disk, a cartridge, a magnetic tape, and/or another type of computer-readable medium, along with a corresponding drive.

Input component 910 may include a component that permits computing device 900 to receive information, such as via user input (e.g., a touch screen display, a keyboard, a keypad, a mouse, a button, a switch, a microphone, and/or the like). Additionally or alternatively, input component 910 may include a sensor for sensing information (e.g., a global positioning system (GPS) component, an accelerometer, a gyroscope, an actuator, and/or the like). Output component 912 may include a component that provides output information from a computing device (e.g., a display, a speaker, one or more light-emitting diodes (LEDs), and/or the like).

Communication interface 914 may include a transceiver-like component (e.g., a transceiver, a separate receiver, and transmitter, etc.) that enables device to communicate with other devices, such as via a wired connection, a wireless connection, or a combination of wired and wireless connections. Communication interface 914 may permit computing device 900 to transmit and/or receive information from another device. For example, communication interface 914 may include an Ethernet interface, an optical interface, a coaxial interface, an infrared interface, a radio frequency (RF) interface, a universal serial bus (USB) interface, a Wi-Fi^{®} interface, a cellular network interface, BLLTETOOTH interface, and/or the like. In non-limiting embodiments or aspects, communication interface 914 does not operate through near field communication. Suitable communication protocols and methods for securing communications between communication interface 914 and a communication interface of another device, such as a computing device (e.g., desktop computer, laptop computer, smartphone, smart watch, PDA, tablet, etc.,) can include encryption, e.g., using a secure socket layer (SSL) (e.g., by using public/private key pairs as are known in the art). Additional security protocols are disclosed in, for example, U.S. Patent Nos. 9,445,264 and 9,463,325, the contents of which are hereby incorporated by reference in their entirety. In non-limiting embodiments described herein, a communication interface 914 is provided on the plunger rod. In non-limiting embodiments or aspects, communication interface 914 is configured to transmit data, but is not configured to receive data transmitted by a computing device, for example a user's smartphone.

A computing device may perform one or more processes described herein. A computing device may perform these processes based on processor 904 executing software instructions stored by a computer-readable medium, such as memory 906 and/or storage component 908, and/or being instructed by a separate computing device. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 906 and/or storage component 908 from another computer-readable medium or from another device via communication interface 914. When executed, software instructions stored in memory 906 and/or storage component 908 may cause processor 904 to perform one or more processes described herein. Additionally or alternatively, hardwired circuitry may be used in place of or in combination with software instructions to perform one or more processes described herein. Thus, embodiments described herein are not limited to any specific combination of hardware circuitry and software.

With reference to FIG. 4, in non-limiting embodiments or aspects, drug delivery device 800 includes a plunger device 810 as described above, including a first switch 820 and second switch 830 in communication with processor 840. Processor 840 is in communication with communication interface 850. The plunger device 810, through communication interface 850, may thus be in communication with a user's computing device 802, such as a smartphone, having its own associated communication interface and processor, as well as memory, storage component, bus, input component, and/or output component.

In non-limiting embodiments or aspects, plunger device 810 is in one-way communication with a user's computing device 802, e.g., the plunger device 810 can only transmit data to the user device 802, and cannot receive data from the user device 802. In non-limiting embodiments or aspects, a processor associated with user device 802 can be programmed or configured, for example based on a mobile application stored in memory and/or storage component, to place communication interface associated with user device 802 in a sleep mode, until communication interface 850 transmits data from plunger device 810.

As described above, in non-limiting embodiments or aspects, upon a user depressing the thumb pad at proximal end of plunger device 810, first switch 820 is activated. As first switch 820 is in communication with processor 840, processor 840 receives a first signal from first switch 820, generates first time data, and transmits, with communication interface 850, first time data, optionally with one or more identifiers (e.g., a device identifier and/or an identifier associated with the communication interface 850), to user device 802. In non-limiting embodiments or aspects, additional data, such as data relating to power level of the battery, a counter value, and/or other data relating to the functioning of drug delivery device 800. In non-limiting embodiments or aspects, first time data and/or one or more identifiers are encrypted before being transmitted to user device 802. In non-limiting embodiments or aspects, processor accepts the data transmitted by communication interface 850 of drug delivery device 800 only if certain conditions are met. In non-limiting embodiments or aspects, the one or more conditions can include that any message received from drug delivery device 800 includes one or more of an identifier associated with the communication interface 850, an identifier associated with the drug delivery device 800, and/or a first identifier (associated, for example, with first signal), associated with activation of the first switch 820. In non-limiting embodiments, the message received from drug delivery device upon activation of first switch 820 is accepted by user device 802 if, following a check, the message is the first transmitted message including the first identifier, indicating that the message has not previously been transmitted by drug delivery device 800. In non-limiting embodiments or aspects, the message is accepted by user device 802 if, following a check, the combination of the first identifier and an identifier associated with the drug delivery device 800 and/or communication interface 850 has not previously been transmitted to user device 802.

In non-limiting embodiments or aspects, drug delivery device 800 continuously transmits data during a drug delivery process. In non-limiting embodiments or aspects, because each message transmitted during the drug delivery process would include the first identifier associated with activation of the first switch 820, user device 802 can be programmed or configured to not accept subsequent message(s) that include the first identifier. In non-limiting embodiments or aspects, once first identifier has been received, user device 802 terminates any connection with plunger device 810, and connection is not reestablished until a second identifier is included in a transmitted message, as described below. In non-limiting embodiments or aspects, user device 802 can, with a processor (e.g, processor 904), generate a first timestamp from the first time data, and may transmit the first timestamp (optionally with one or more identifiers associated with drug delivery device 800 as described herein) to healthcare system 804. In non-limiting embodiments or aspects, when first timestamp is transmitted to healthcare system 804, user device 802 terminates any connection with plunger device 810.

As also described above, upon reaching the end of the drug delivery process, the second switch 830 is activated. As second switch 830 is in communication with processor 840, processor 840 receives a signal from second switch 830 generates second time data, and transmits, with communication interface 850, second time data, optionally with one or more identifiers (e.g., a device identifier and/or an identifier associated with the communication interface 850), to user device 802. In non-limiting embodiments or aspects, second time data and/or one or more identifiers are encrypted before being transmitted to user device 802. As discussed above, in non-limiting embodiments or aspects, processor accepts the data transmitted by drug delivery device 800 only if certain conditions are met. In non-limiting embodiments or aspects, the one or more conditions can include that any message received from drug delivery device 800 includes one or more of an identifier associated with the communication interface 850, an identifier associated with the drug delivery device 800, and/or a second identifier associated with activation of second switch 830. In non-limiting embodiments, the message received from drug delivery device upon activation of second switch 830 is accepted by user device 802 (and, in non-limiting embodiments, communication is reestablished between drug delivery device 800 and user device 802) because such message is the first transmitted message including the second identifier, indicating that the message has not previously been transmitted by drug delivery device 800. In non-limiting embodiments or aspects, user device 802 can, with a processor (e.g, processor 904), generate a second timestamp from the second time data, and may transmit the second timestamp (optionally with one or more identifiers associated with drug delivery device 800 as described herein) to healthcare system 804. In non-limiting embodiments or aspects, when second timestamp is transmitted to healthcare system 804, user device 802 terminates any connection with plunger device 810.

In non-limiting embodiments or aspects, based at least partially on and at least partially in response to receipt of the data (first time data and/or second time data), user device 802 can, with a processor (e.g, processor 904), generate a final timestamp. For example, first time data and second time data generated by drug delivery device 800 may include only absolute time information, from which user device 802 may generate final timestamp data (e.g., a time at which the drug delivery process began and ended) and/or duration data (e.g., a duration of the drug delivery process).

In non-limiting embodiments or aspects, user device 802 transmits final timestamp and/or duration data to healthcare system 804 (optionally with one or more identifiers associated with drug delivery device 810 as described herein). In non-limiting embodiments or aspects, healthcare system can verify the timestamp data and/or duration data, for example by comparing the timestamp data and/or duration data to predetermined ranges/values for each. In non-limiting embodiments or aspects, data falling outside of the predetermined ranges can be marked for follow up, or a message can be sent to user device 802 noting the issue.

In non-limiting embodiments or aspects, healthcare system 804 may be in communication with one or more additional healthcare systems 804. For example, one healthcare system may be maintained by the device manufacturer, and may be in communication with a healthcare system maintained by a study sponsor, pharmaceutical manufacturer, or the like. In non-limiting embodiments or aspects, timestamp (first timestamp, second timestamp, and/or final timestamp) and/or duration data, together with one or more identifiers (e.g., a device identifier and/or an identifier associated with the communication interface 850), may be transmitted from device manufacturer system to study sponsor or pharmaceutical manufacturer system, where one or more databases may store data associating the one or more identifiers (e.g., a device identifier and/or an identifier associated with the communication interface 850) with a patient identifier, allowing for association of timestamp and/or duration data with the patient.

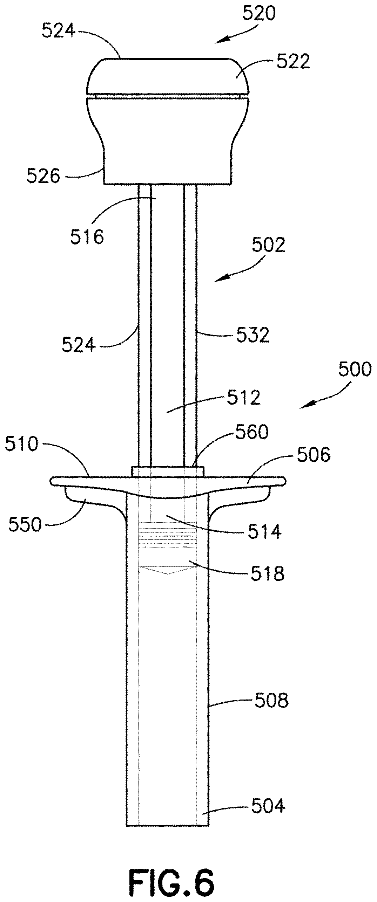

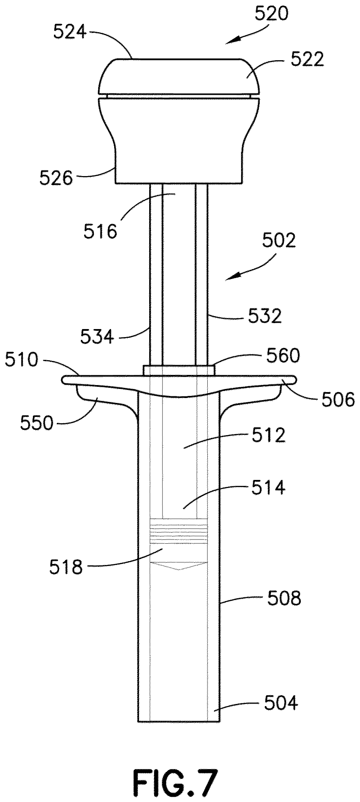

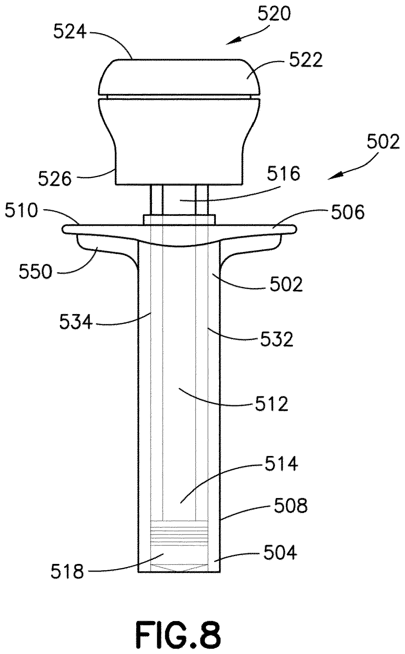

With reference to FIGS. 6-10, according to one non-limiting embodiment or aspect of the present disclosure, a drug delivery device 500 with a plunger device 502 is shown and described. This drug delivery device 500 may be similar to the drug delivery device 100 described above. While a manual syringe is exemplified in FIGS. 6-8, those of skill in the art will appreciate that the components of the plunger device 502 can be utilized in any type of drug delivery device, including autoinjectors, wearable injectors, pump devices, and the like. The drug delivery device 500 exemplified in FIGS. 6-8 includes a distal end 504, proximal end 506, and barrel 508 therebetween defining an interior configured to hold one or more medicaments.

Drug delivery device 500 can be formed of any suitable material, including glass or plastics. Barrel 508 can be coated with any known coating(s), as appropriate to decrease friction between components and/or reduce interactions between the material of barrel 508 and any medicaments held within drug delivery device 500, including polydimethylsiloxane-based coatings that are optionally crosslinked. Coatings for decreasing friction between a stopper and sidewall and/or for reducing interactions between the sidewall and the medicament, for example siloxane-based coatings, are known to those of skill in the art, and are described in, for example, U.S. Patent Application Publication Nos. 2014/0221934 and 2014/0110297, the contents of which are incorporated herein by reference in their entirety.

In non-limiting embodiments or aspects, drug delivery device 500 includes a needle (not shown) at a distal end thereof. Suitable needles can be of any useful gauge, can be formed of any suitable material, and can be fixed to distal end 504 of drug delivery device 500, or can be removable. Drug delivery device 500 can include any number of suitable safety features, including a rigid needle shield (RNS) for protecting any needles that may be included, a cap, and/or a safety device, such as those shown in FIGS. 1A and 1B. RNSs are known in the art, and can include an inner, elastomeric portion that encases an injection needle, and an outer, rigid plastic portion. Suitable cap assemblies are described in, for example, International Patent Application Publication No. WO 2020/173998, the content of which is incorporated herein by reference in its entirety. Suitable safety devices can include those that prevent reuse of the syringe, those that shield any needles before and/or after delivery, and the like, as are known to those of skill in the art. For example, safety devices are described in U.S. Patent No. 10,702,663, and are available commercially from Becton Dickinson and Company under the tradename UltraSafe^{™}. As will be described, in non-limiting embodiments or aspects, the safety device can interact with plunger device 502 to indicate end of drug delivery.

Returning to FIGS. 6-8, drug delivery device 500 can include at proximal end 506 thereof one or more finger flange(s) 510, for assisting a user in gripping drug delivery device 500. As will be described, in non-limiting embodiments or aspects, one or more finger flange(s) 510 can interact with plunger device 502 to indicate end of drug delivery.

Referring to FIGS. 6-8, plunger device 502 can include a plunger rod 512 extending between a distal end 514 and a proximal end 516. Distal end 514 of plunger rod 512 can be configured to interact, detachably or fixedly, with a stopper 518. Stopper 518 can be formed of any material, such as elastomeric materials, known to those in the art to be suitable, such as plastics, natural rubbers, synthetic rubbers, and the like. In non-limiting embodiments or aspects, a useful stopper is formed of a natural or synthetic rubber. In non-limiting embodiments or aspects, the rubber is butyl rubber (IIR), isoprene rubber (IR), butadiene rubber (BR), styrene-butadiene rubber (SBR), ethylene-propylene rubber (EPM), ethylene-propylene-diene rubber (EPDM), chlorosulphonated polyethylene (CSM), ethylene-vinyl acetate copolymer (EVA), styrene-isoprene rubber (SIR), thermoplastic elastomers, and/or natural rubbers. In non-limiting embodiments or aspects, the stopper can be formed of an elastomeric copolymer, including, without limitation, thermoplastic elastomers, thermoplastic vulcanizates, styrene copolymers such as styrene-butadiene (SBR or SBS) copolymers, styrene-isoprene (SIS) block polymers, or styrene-isoprene/butadiene (SIBS).

Stopper 518 can optionally be coated with any known coating(s), as appropriate, including polydimethylsiloxane-based coatings that are optionally crosslinked. Coatings for decreasing friction between a stopper and sidewall and/or for reducing interactions between the material of the stopper and the medicament, for example siloxane-based coatings, are known to those of skill in the art, and are described in, for example, U.S. Patent Application Publication Nos. 2014/0221934 and 2014/0110297, the contents of which are incorporated herein by reference in their entirety.

Plunger device 502 can include at proximal end 516 thereof a connection module 520, having a housing 522 having a proximal end 524 and a distal end 526 defining an interior. Connection module housing 522 can be formed of any suitable material, including plastics.

Referring to FIG. 9, shown are non-limiting embodiments of the interior of connection module 520. Connection module 520 can include a microcontroller 528 including one or more processors (as will be described in greater detail below). In non-limiting embodiments or aspects, the microcontroller 528 can be circuit board(s) including printed circuit boards, flexible substrates for printed electronics, and the like as are known in the art.

In non-limiting embodiments or aspects, connection module 520 includes an energy source, such as a battery 530. In non-limiting embodiments or aspects, battery 530 can be disposable or rechargeable, depending on whether plunger device 502 is a single-use device, or is reusable. In non-limiting embodiments in which the plunger device 502 is a single-use device, housing 522 can be configured to prevent separation of any portions of housing 522, to prevent a user from accessing interior of housing 522. In non-limiting embodiments or aspects in which plunger device 502 is a single-use device, housing 522 may be openable to allow for a user to remove one or more components (e.g., battery 530 and/or microcontroller 528) for appropriate disposal. In non-limiting embodiments in which plunger device 502 is a reusable device, housing 522 can be configured to be openable to allow for maintenance and/or replacement of battery 530. In non-limiting embodiments, housing 522 is openable only with use of a particular tool. In non-limiting embodiments or aspects in which plunger device 502 is a reusable device, battery 530 may be a rechargeable battery, and housing 522 may include one or more ports to allow for the battery 530 to be recharged.

With reference to FIGS. 6-8, the plunger rod 512 may include at least one tracking element 532, 534 positioned thereon. In one embodiment, the plunger rod 512 may include two tracking elements 532, 534. In one embodiment, the tracking elements 532, 534 may be conductive strips that are adhesively or integrally formed on the plunger rod 512. By using these tracking elements 532, 534, the plunger rod 512 is used to track and transmit the start of injection with the plunger device 502, the progression of injection with the plunger device 502, and the completion of the injection with the plunger device 502.

As shown in FIGS. 6-8, the tracking elements 532, 534 may be positioned parallel to one another or, in an alternative embodiment, substantially parallel to one another. Longitudinal axes of the tracking elements 532, 534 extend along a longitudinal axis of the plunger rod 512. In one example, proximal ends of the tracking elements 532, 534 may be operatively connected to the battery 530, which may be a constant voltage power supply battery. The distal ends of the tracking elements 532, 534 are not connected to one another or the battery 530.

With reference to FIG. 9, according to one non-limiting embodiment or aspect, the housing 522 of the connection module 520 may also include a Bluetooth low energy (BLE) module 536, a current sensing device 538, and a force sensor 540. The force sensor 540 may be configured to sense the initiation or start of injection when a user presses on the connection module 520.

With reference to FIGS. 6-8 and 10, according to a non-limiting embodiment or aspect of the present disclosure, a backstop 550 may be provided on the finger flange 510 of the drug delivery device 500. The backstop 550 may be operatively connected to the finger flange 510 and/or the barrel 508 of the drug delivery device 500 via an adhesive, by welding, by integral molding, or any other connection method. The backstop 550 may define an opening 552 that receives the barrel 508 of the drug delivery device 500. In one example, an inner surface of the opening 552 may include a conductive strip 554 that extends around at least a portion of the circumference of the opening 552. Since the conductive strip 554 is not provided on the plunger rod 512, the conductive strip 554 may be understood to be "external" to the plunger rod 512. In another example, the conductive strip 554 extends around the entire circumference of the opening 552. In another example, the conductive strip 554 may include two separate strips that are included on separate portions of the circumference of the opening 552. It is to be understood that the conductive strip 554, whether a single strip or multiple strips, should at least cover a portion of the circumference of the opening 552 such that the conductive strip 554 makes contact with the tracking elements 532, 534 on the plunger rod 512. In one example, the conductive strip 554 may be positioned on the upper lip of the opening 552. In another example, the conductive strip 554 may be positioned on the lower lip of the opening 552. In yet a further example, a conductive strip may be positioned on the upper lip and a separate conductive strip may be positioned on the lower lip of the opening 552.

During operation of the drug delivery device 500, as the plunger rod 512 is pushed towards the distal end 504 of the drug delivery device 500, the tracking elements 532, 534 on the plunger rod 512 come into electrical contact with the conductive strip 554 on the backstop 550. The electrical contact between the tracking elements 532, 534 on the plunger rod 512 and the conductive strip 554 closes an electrical circuit established with the battery 530 and an electrical current can flow through the tracking elements 532, 534. The moderate conductivity of the tracking elements 532, 534 and the conductive strip 554 ensures that the power supply (i.e., battery 530) is not short-circuited. The electrical circuit established between the tracking elements 532, 534 and the conductive strip 554 may be identified by the current sensing device 538 that is in communication with the battery 530, which may send a signal to the BLE module 536 to identify the position of the plunger rod 512 within the drug delivery device 500. As the injection proceeds, the electrical resistance of the load experienced by the battery 530 decreases and the electrical current flowing through the electrical circuit increases. This increase in current may be sensed by the current sensing device 538 and correlated to the position of the plunger rod 512 relative to the backstop 550 and, therefore, can be used to monitor the injection progression of the plunger rod 512. When the injection is completed, the electrical resistance is minimal and the electrical current is maximal. This maximum electrical current serves to indicate the injection completion. The BLE module 536 enables transmission of this monitoring to a distant or remote device (within BLE range), such as a smartphone or remote computer. A smartphone application may be used to display injection data (including injection start, injection progression, and injection completion), as well as keep a record of previous injections, plus any other feature that is desirable for a use monitoring situation. The current sensing device 538 may be configured to receive signals regarding the resistance of the electrical circuit established between the tracking elements 532, 534 and the conductive strip 554. The current sensing device 538 may receive these signals during the entire injection procedure of the drug delivery device 500. It is also contemplated that capacitive sensing could also be used in place of the resistive/current sensing identified above.

As shown in FIGS. 6-8, according to one non-limiting embodiment or aspect of the present disclosure, a ring 560 may be placed around the plunger rod 512, in which an inner circumference of the ring 560 may be covered with a conductive strip that can replace the backstop 550. This ring 560 may be kept in contact with the upper surface of the finger flange 510 to maintain the position of the ring 560 relative to the drug delivery device 500. The ring 560 may define an inner cavity or opening that permits the plunger rod 512 to pass therethrough. Similar to the conductive strip 554 on the backstop 550, the conductive strip on the ring 560 may establish an electrical contact with the tracking elements 532, 534 of the plunger rod 512 to complete the electrical circuit to monitor the injection procedure.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A plunger device for a drug delivery device, comprising:
a plunger rod having a proximal end and a distal end configured to be attached to a stopper;
at least one tracking element operatively connected to an outer surface of the plunger rod, the at least one tracking element extending along a longitudinal axis of the plunger rod;
at least one power supply operatively connected to the at least one tracking element; and
at least one current sensing device operatively connected to the at least one power supply and configured to detect an electrical contact established between the at least one tracking element and a conductive element external to the plunger rod.

2. The plunger device of claim 1, wherein the plunger rod is configured to be used with a manual syringe.

3. The plunger device of any of claims 1-2, wherein the proximal end of the plunger rod comprises a housing comprising a proximal end configured to be contacted by a user's thumb during drug delivery, a distal end attached to the proximal end of the plunger rod, and a sidewall therebetween defining an interior.

4. The plunger device of any of claims 1-3, wherein the proximal end of the housing is displaceable relative to the distal end of the housing when a distally-directed force is applied by the user during drug delivery.

5. The plunger device of claims 1-4, wherein the at least one tracking element comprises two tracking elements positioned on the outer surface of the plunger rod opposite one another.

6. The plunger device of any of claims 1-5, wherein the at least one tracking element is a conductive strip.

7. The plunger device of any of claims 1-6, wherein the at least one power supply comprises a battery.

8. The plunger device of any of claims 1-7, further comprising a force sensor configured to identify a pressure applied to the proximal end of the plunger rod.

9. The plunger device of any of claims 1-8, wherein the at least one tracking element extends from the proximal end of the plunger rod to the distal end of the plunger rod.

10. The plunger device of any of claims 1-9, further comprising a communication interface for issuing a signal when the electrical contact has been established between the at least one tracing element and the conductive element external to the plunger rod.

11. A drug delivery device comprising:
a proximal end;
a distal end; and
a barrel therebetween defining an interior configured to receive a medicament, and
the plunger device of claim 1 received at least partially within the interior.

12. The drug delivery device of claim 11, further comprising a backstop operatively connected to a finger flange provided on the proximal end of the drug delivery device, the backstop comprising the conductive element external to the plunger rod.

13. The drug delivery device of any of claims 11-12, further comprising a ring operatively connected to the drug delivery device and configured to receive the plunger rod, the ring comprising the conductive element external to the plunger rod.

14. A method of monitoring an injection procedure of a drug delivery device, comprising:
providing a drug delivery device as recited in claim 11;
receiving, with the at least one current sensor device, a first signal indicating that the electrical contact between the at least one tracking element and the conductive element external to the plunger rod has been established;
transmitting, with at least one communication interface, the first signal to a remote device;
receiving, with the at least current sensing device, a second signal indicating a resistance of an electrical circuit established between the at least one tracking element and the conductive element external to the plunger rod; and
transmitting, with the at least one communication interface, the second signal to the remote device.

15. The method of claim 14, wherein the at least one current sensing device is configured to receive signals of the resistance of the electrical circuit during the entire injection procedure.
